# FASCICULE DE BREVET EUROPEEN

(11) **EP 3 834 885 B1**
(45) Date de publication et mention de la délivrance du brevet: **29.06.2022**
(21) Numéro de dépôt: 20211920.2
(22) Date de dépôt: 04.12.2020
(51) Int. Cl.: A61N 5/06, A61N 1/05, A61N 1/36

(54) **DISPOSITIF D'ILLUMINATION IMPLANTABLE DANS UN ÊTRE VIVANT**
IN EIN LEBEWESEN IMPLANTIERBARE BELEUCHTUNGSVORRICHTUNG
ILLUMINATION DEVICE IMPLANTABLE IN A LIVING BEING

(30) Priorité: 12.12.2019 FR 1914302
(43) Date de publication de la demande: 16.06.2021
(73) Titulaire: Commissariat à l'Energie Atomique et aux Energies Alternatives, 75015 Paris (FR)
(72) Inventeur: CHABROL, Claude, 38054 GRENOBLE cedex 09 (FR); CAILLAT, Patrice, 38054 GRENOBLE cedex 09 (FR)
(74) Mandataire: INNOV-GROUP

(56) Documents cités:
- EP-A1- 3 539 449
- WO-A1-2017/103380
- US-A1- 2019 168 022

## Description

### Domaine technique de l'invention

La présente invention se rapporte à un dispositif d'illumination destiné à être implanté au moins en partie dans un être vivant pour illuminer, de manière localisée, au moins une zone de l'être vivant.

### Etat de la technique

Pour le traitement de certaines pathologies d'un être vivant, il a été imaginé d'aller stimuler de manière optique une zone interne d'un être vivant. Pour cela, il a été proposé des dispositifs qui comportent une source de lumière et qui sont implantés au moins en partie ou totalement dans l'être vivant pour aller illuminer la zone souhaitée.

On s'est notamment aperçus de l'intérêt de tels dispositifs pour illuminer/irradier de manière optique certaines zones du cerveau humain.

Cependant, compte-tenu des risques liés à l'implantation d'un tel dispositif dans le cerveau, on comprend qu'un tel dispositif doive être parfaitement conçu.

La demande de brevet US2017281928A1 et le brevet US10213596B2 décrivent des dispositifs d'illumination implantables comprenant un générateur d'impulsions (IPG pour "Implantable Pulse Generator") qui alimente une source lumineuse et une sonde comportant un guide lumière chargé d'amener un faisceau lumineux vers la zone à traiter.

D'autres solutions ont également été proposées dans les documents référencés US2019/168022A1, EP3539449A1 et WO2017/103380A1.

Ces solutions ne répondent pas à certains ou à tous les critères suivants :
- Illumination sur une longueur adaptable de 2 à 50mm
- Compatibilité d'une source d'illumination avec des générateurs de type IPG disponibles sur le marché (c'est-à-dire sans modification "hardware") ;
- Modularité (possibilité de stimuler de manière électrique et/ou optique, choix de plusieurs longueurs d'ondes d'illumination...) ;

Par ailleurs, il est connu que la courbe d'efficacité du traitement suit une courbe en cloche en fonction de la durée du traitement et de la puissance optique injectée.

A l'heure actuelle, il n'existe pas de solutions simples et compactes permettant de mesurer l'efficacité d'un tel traitement.

Le but de l'invention est de proposer un dispositif implantable de stimulation optique configuré pour remplir un ou plusieurs des objectifs visés ci-dessous, c'est-à-dire :
- Compacité et adaptation à une chirurgie de stimulation profonde (DBS pour "Deep Brain Stimulation") ;
- Compatibilité avec des générateurs de type IPG ("Implantable Pulse Generator") existants ;
- Modularité et polyvalence (stimulation électrique et/ou optique, choix de la longueur d'illumination ;
- Efficacité thérapeutique permise par un suivi des paramètres biologiques par caractérisation du milieu environnant (méthode optique, thermique, électrique) ;
- Surveillance du bon fonctionnement de la sonde ;

Par ailleurs la solution de l'invention pourra permettre de remplir les objectifs suivants :
- Évaluation de la pénétration de la lumière dans les tissus afin d'ajuster la dose appliquée au patient ;
- Stimulation multi-longueurs d'ondes ;
- Assistance à la mise en place chirurgicale en enregistrant les paramètres d'absorption/diffusion pendant l'insertion de la sonde dans les tissus biologiques ;
- Surveillance de l'évolution des paramètres optiques des tissus environnants par différentes méthodes (DRS pour "Diffuse Reflectance Spectroscopy", Fluorescence, pics d'absorption de molécules biologiques, suivi thermique) ;

La solution de l'invention sera mise en œuvre à base de matériaux reconnus comme biocompatibles.

### Exposé de l'invention

Ce but est atteint par un module à stimulation optique à intégrer dans une sonde implantable dans un être vivant pour illuminer de manière localisée une zone dudit être vivant, ledit module comportant :
- Un boîtier au moins en partie transparent,
- Un bloc électronique hermétique placé dans ledit boîtier et comportant un volume interne logeant une source lumineuse, une unité de commande et de traitement, un premier photodétecteur et un deuxième photodétecteur, un écran opaque séparant son volume interne en un premier espace contenant la source lumineuse et le deuxième photodétecteur, et un deuxième espace contenant le premier photodétecteur,
- Un dispositif de connexion pour raccorder ledit module à une unité centrale d'alimentation et de commande.

Selon une particularité, le module comporte un filtre optique de sélection de longueurs d'ondes appliqué audit premier photodétecteur.

Selon une autre particularité, le module peut comporter au moins un guide d'ondes agencé sur le premier photodétecteur en vue de limiter toute lumière parasite et d'optimiser une captation de photons.

Selon une autre particularité, le module comporte un hublot transparent, choisi pour capter des signaux de longueur d'onde supérieure à 4µm.

Selon une autre particularité, le bloc électronique comporte au moins un substrat comportant deux faces opposées, lesdites deux sources lumineuses étant montées sur une seule des deux faces dudit substrat.

Selon une autre particularité, le bloc électronique peut comporter un capot hermétique adapté sur le substrat et formant ledit volume interne du bloc électronique.

Selon une autre particularité, le boîtier du module peut comporter une bague fermée à ses deux extrémités par deux bouchons, lesdits deux bouchons portant des moyens de support du bloc électronique logé dans le boîtier.

Selon une autre particularité, le module peut comporter des électrodes de stimulation électrique sur la surface latérale de sa bague.

Selon une autre particularité, le module peut comporter un matériau d'enrobage injecté dans son boîtier autour du bloc électronique hermétique.

L'invention concerne également une sonde implantable dans un être vivant destinée à être connectée électriquement à une source d'alimentation électrique et présentant une architecture allongée, ladite sonde comportant plusieurs modules à stimulation optique tels que définis ci-dessus, lesdits modules étant juxtaposés le long de la sonde et séparés entre eux d'une distance non nulle, ladite sonde comprenant un matériau d'enrobage venant combler l'espace entre deux modules adjacents.

L'invention concerne également un dispositif d'illumination implantable, destiné à être implanté dans un être vivant pour illuminer de manière localisée une zone dudit être vivant, ledit dispositif comportant une source d'alimentation électrique comportant plusieurs voies d'alimentation électrique en parallèle et une sonde connectée électriquement à la source d'alimentation électrique et présentant une architecture allongée entre une extrémité proximale et une extrémité distale, ledit dispositif comprenant une sonde telle que définie ci-dessus.

Selon une particularité, la source d'alimentation est un générateur d'impulsions implantable.

Selon une autre particularité, le dispositif comporte des moyens de détermination d'une dose de lumière à appliquer en fonction de signaux reçus en provenance d'un ou plusieurs premiers photodétecteurs des modules.

### Brève description des figures

D'autres caractéristiques et avantages vont apparaître dans la description détaillée qui suit faite en regard des dessins annexés dans lesquels :
- La figure 1 représente un exemple de réalisation du dispositif conforme à l'invention ;
- Les figures 2A et 2B illustrent différents types de signaux de courant ou tension de stimulation pouvant être émis par le générateur employé dans le dispositif de l'invention ; La figure 2C illustre un exemple de signaux pulsés servant à mettre en œuvre un monitoring d'impédance ;
- Les figures 3 et 4 représentent de manière schématique l'architecture du dispositif conforme à l'invention, la figure 4 étant un zoom sur la sonde du dispositif ;
- Les figures 5A à 5C et 6A à 6C représentent, de manière schématique, un module conforme à l'invention, respectivement en vue de côté, en vue de dessus et vu suivant son axe, selon deux réalisations particulières distinctes ;
- Les figures 7A à 7G représentent plusieurs réalisations du module, en vue de côté et vue suivant son axe ;
- Les figures 8A, 8B et 8C représentent de manière schématique un principe de connexion d'un module conforme à l'invention sur un câble, respectivement en vue de côté, en vue de dessus et vu suivant une coupe transversale ;
- La figure 9 représente de manière schématique l'architecture fonctionnelle d'un module conforme à l'invention ;
- La figure 10 illustre le principe d'adressage d'un module à partir du générateur IPG ;
- La figure 11 représente l'architecture électronique du module et le principe de connexion électrique et d'adressage du module ;
- La figure 12A représente des chronogrammes illustrant le principe d'un premier mode de fonctionnement du dispositif, correspondant à une stimulation par l'alimentation des diodes lumineuses ;
- La figure 12B représente des chronogrammes illustrant le principe d'un deuxième mode de fonctionnement du dispositif, correspondant à une lecture des signaux mesurés par un photodétecteur du module après commande des diodes lumineuses ;
- La figure 13 illustre le principe de mesure mis en œuvre pour ajuster la dose optique à l'aide du dispositif de l'invention ;
- Les figures 14A et 14B illustrent différentes configurations d'illumination permises par le dispositif de l'invention ;

### Description détaillée d'au moins un mode de réalisation

L'invention concerne un dispositif 1 implantable d'illumination cérébrale profonde (DBS - "Deep Brain Stimulation"). Ce dispositif permet notamment de réaliser une illumination localisée (par exemple dans le proche infra-rouge ou avec toute autre longueur d'onde selon le traitement envisagé - traitement type neuroprotection, optogénétique) des tissus cibles (par exemple SNc, hippocampe, striatum...) tout en minimisant les risques médicaux lors de l'implantation. Ce dispositif peut servir en particulier dans le traitement de maladies neurodégénératives telles que la maladie de Parkinson, Alzheimer, Huntington...

On verra que le dispositif 1 peut éventuellement intégrer des solutions proposant d'autres modes de stimulations (électrique notamment).

L'illumination des tissus peut avoir différents objectifs selon l'application : neuroprotection, optogénétique, stimulation... Plusieurs cibles sont concernées, par exemple : la substance noire compacta (SNc) qui dégénère dans la maladie de Parkinson, l'hippocampe, principal noyau mis en cause dans le début de la maladie d'Alzheimer, le striatum pour la maladie d'Huntington. L'illumination peut être apportée directement dans les tissus (illuminateur bidirectionnel, cette stratégie chirurgicale présente des risques de lésions supplémentaires) ou en choisissant des trajectoires passant par les ventricules (cavités permettant la circulation du liquide céphalorachidien LCR) et en contact avec les structures à traiter (illuminateurs directifs).

Par ailleurs, le dispositif permet également d'effectuer des mesures en vue de mettre en œuvre différents diagnostics et suivis sur les tissus environnants. Le dispositif permet de surveiller le bon fonctionnement individuel des sources de lumière pour garantir un bon traitement.

Le dispositif 1 de l'invention comporte une source d'alimentation électrique. Cette source d'alimentation est avantageusement composée d'un générateur d'impulsions implantable (appelé couramment IPG pour "Implantable Pulse Generator"), référencé IPG sur les dessins.

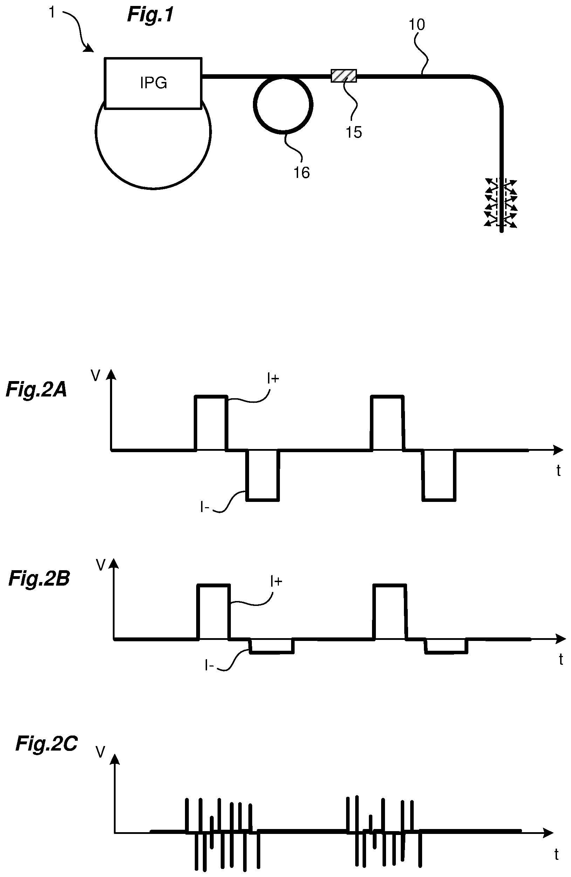

De manière connue, un générateur d'impulsions implantable IPG comporte principalement une carte électronique et une batterie, rechargeable ou non. La carte électronique comporte un microcontrôleur chargé de gérer le fonctionnement du générateur. Le générateur IPG peut notamment être programmé pour fournir des impulsions dites bipolaires, telles que celles représentées sur les figures 2A et 2B. Sur les figures 2A et 2B, le motif comporte ainsi une impulsion carrée positive I+ et une impulsion carrée négative I-, séparées entre elles d'un temps mort. Pour équilibrer les charges électriques injectées dans les tissus, les deux impulsions peuvent être symétriques, comme illustré sur la figure 2A, ou modulées en amplitude et en durée, comme illustré sur la figure 2B, avec la même quantité de charge injectée pour les deux impulsions (t x i x V avec t durée de l'impulsion, i le courant et V la tension). Le générateur comporte n voies, avec n supérieur ou égal à 2, par exemple compris entre 4 et 12 voies.

La figure 2C représente les signaux émis par le générateur IPG qui permettent de réaliser une surveillance de l'impédance de la sonde 10 qui lui est connectée. Ces signaux peuvent être codés afin d'adresser individuellement chaque fonction de chacun des blocs électroniques 3.

Le dispositif 1 comporte ensuite une sonde 10 implantable connectée au générateur.

Cette sonde 10 implantable se présente sous la forme d'une tige allongée souple. La sonde 10 présente avantageusement une section transversale circulaire. A titre d'exemple, le diamètre de la section de la sonde peut aller de 1 à 3mm, préférentiellement 1.3mm pour être compatible avec les outils standards utilisés en DBS.

Le dispositif 1 comporte des moyens de connexion électrique permettant de connecter la sonde 10 au générateur IPG par son extrémité proximale, par l'intermédiaire d'un connecteur 15 et d'une extension 16. À l'extrémité distale, la sonde 10 présente avantageusement une forme atraumatique 100 (par exemple avec une forme oblongue ou sphérique).

Selon l'invention, sur au moins une partie de sa longueur, la sonde 10 intègre plusieurs modules M de stimulation optique juxtaposées formant une barrette. Cette barrette est avantageusement située à proximité de l'extrémité distale de la sonde 10.

Le nombre de modules de la sonde 10 peut être adapté selon la pathologie à traiter et selon la taille du tissu traité (pouvant aller par exemple de 5 à 50mm de long).

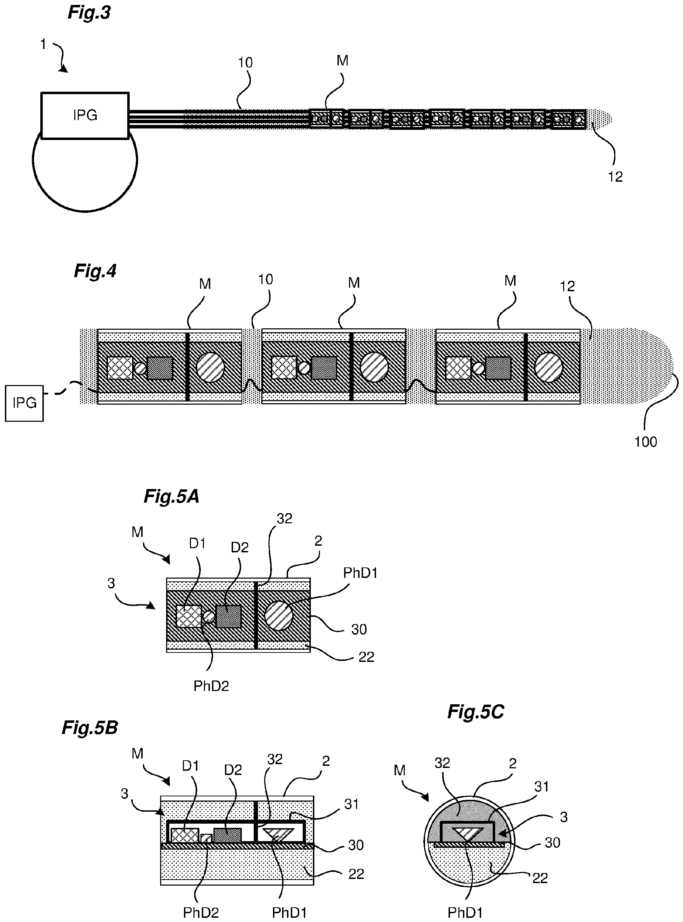

En référence aux figures 3 et 4, les modules M sont connectés entre eux de manière à former une guirlande et maintenus entre eux par un matériau d'enrobage 12 de type silicone, polyuréthane ou époxy, le matériau pouvant être choisi notamment en fonction de la rigidité visée pour l'application. Chaque module M est relié au générateur IPG par une liaison point à point. On verra ci-dessous que la liaison point à point intègre plusieurs conducteurs nécessaires au fonctionnement du module.

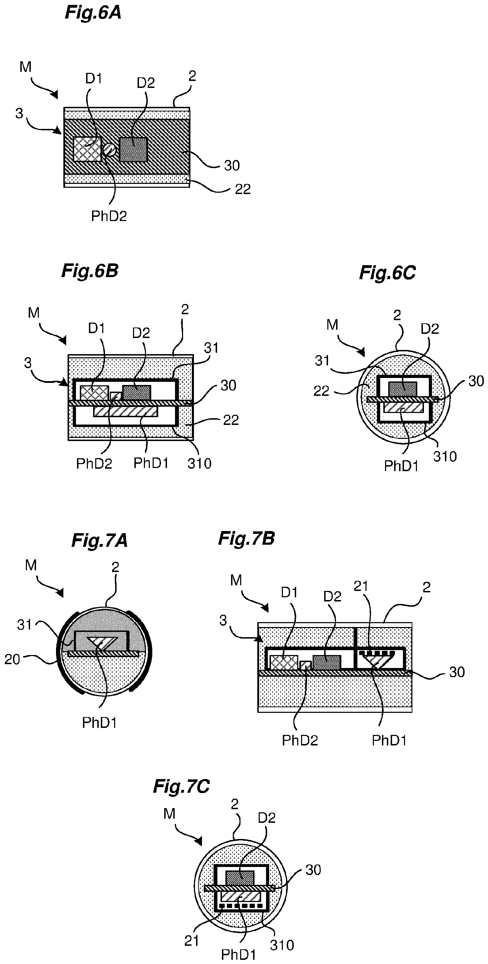

En référence aux figures 5A à 5C et 6A à 6C, chaque module M de stimulation optique peut comporter un boîtier 2. Le boîtier 2 peut comporter une bague réalisée dans un matériau transparent et biocompatible tel que le saphir ou la silice et fermée à ses deux extrémités par des bouchons (non représentés), de manière à former un cylindre creux intégrant un bloc électronique muni notamment d'un bloc optique. Le cylindre peut présenter un diamètre externe de 1300µm.

Les deux bouchons servent au bon positionnement du bloc électronique et facilitent le remplissage de la cavité par un matériau d'enrobage 22 transparent ou diffusant selon l'objectif d'uniformité recherché (type silicone, polyuréthane ou époxy). Une colle époxy pourra être sélectionnée avec un indice optique limitant les pertes par réflexion, et pourra également être chargée afin d'améliorer le transfert thermique et l'étanchéité, et répondre aux contraintes de dilatation pendant la production. Dans la sonde, chaque module M est ainsi indépendant de chaque autre module et présente sa propre architecture électronique, c'est-à-dire son bloc électronique et ses contacts électriques de connexion, et sa propre architecture mécanique composée de la bague et des bouchons. Il faut noter que l'herméticité du bloc électronique est obtenue au niveau de chaque module, rendant le module facilement intégrable dans une sonde lors de sa fabrication.

Le bloc électronique comporte des moyens de stockage d'énergie électrique type micro-batterie, capacité MOS ou condensateur C1, C2 pour alimenter en électricité le bloc électronique 3 entre deux pulses d'alimentation fournis par le générateur IPG.

Le bloc électronique de chaque module M comporte une ou plusieurs sources lumineuses. Les sources lumineuses peuvent être de type LED, OLED, µLED, VCSEL, LASER, QCL...). Les sources lumineuses peuvent être monolithiques (de type LED) ou matricielle (par hybridation de matrice de µLED de différentes longueurs d'ondes).

Les sources lumineuses peuvent fonctionner à une tension inférieure à quelques volts (2V par exemple pour les LED), avec des courants pouvant aller de 2mA à 25mA. Le générateur IPG peut pour sa part fournir une tension de 15V et des courants allant de de 25 à 50mA, ce qui permet par exemple l'alimentation de plusieurs composants en série (exemple jusqu'à sept diodes de 2V).

De manière non limitative, comme représenté sur les figures annexées, chaque module M comporte deux diodes lumineuses D1, D2. Les deux diodes lumineuses D1, D2 peuvent être identiques et émettre à une même longueur d'onde. Mais il est aussi possible de prévoir d'utiliser deux diodes qui émettent à deux longueurs d'onde distinctes, par exemple à 670nm pour l'une et 810nm pour l'autre dans l'objectif d'une neuroprotection.

Dans chaque module M, d'autres sources lumineuses que celles utilisées pour la stimulation optique et de longueur d'onde adaptée peuvent être intégrées afin de permettre des mesures des tissus environnants par mesure de lumière rétrodiffusée (DRS, pics absorption spécifiques à une molécule biologique (hémoglobine vs oxyhémoglobine, glucose...). Les détecteurs intégrés aux modules devront être adaptés aux longueurs d'ondes d'émission des sources.

Dans chaque module M, un capteur de température (par exemple à technologie CMOS) pourra être intégré au module électronique afin de permettre un suivi du fonctionnement du dispositif (sécurité thermique en cas d'échauffement) et permettre un suivi de la température locale du tissu environnant (dépendant de la quantité de protéine Tau dans le cas de la maladie d'Alzheimer par exemple). Un élément conducteur thermique (par exemple un doigt en matériau conducteur agencé entre le capteur et le boîtier du module) peut être intégré au module M pour faciliter et fiabiliser la prise de température.

L'illumination est réalisée selon plusieurs directions transversales (notamment radiales pour une sonde à section circulaire) par rapport à l'axe de la sonde. Selon les modules employés, il est possible d'illuminer selon différentes directions, sur toute la périphérie de la sonde ou sur une plage angulaire déterminée plus étroite. Il sera notamment possible d'employer des modules à illumination bidirectionnelle (par exemple 2x110°, angle d'émission standard pour des LED) ou des modules à illumination plus directive (par exemple 1x110°).

Le bloc électronique 3 peut comporter un premier photodétecteur PhD1. Ce premier photodétecteur PhD1 est fixé sur le substrat 30 du bloc électronique 3 et est positionné pour détecter les signaux optiques transmis par les tissus environnants. Ce premier photodétecteur PhD1 peut être par exemple une photodiode grande surface hybridée ou une matrice de détecteurs CMOS.

Le bloc électronique 3 peut comporter un deuxième photodétecteur PhD2. Ce deuxième photodétecteur PhD2 est fixé sur le substrat 30 du bloc électronique et est positionné pour surveiller le bon fonctionnement des diodes lumineuses. Ce deuxième photodétecteur PhD2 peut être par exemple une photodiode à technologie CMOS ou une photodiode hybridée.

De manière non limitative, le bloc électronique 3 peut également comporter tout autre capteur physique participant au suivi biologique et physiologique des tissus environnants, par exemple capteur de température, optique, électrique dans un but de contrôle de l'évolution du tissu environnant, de l'ajustement du traitement pour chaque patient et chaque zone d'illumination (profondeur de pénétration de la lumière localement dans le tissu environnant) et de sécurité du dispositif.

De manière non limitative, le bloc électronique 3 peut comporter un substrat 30 présentant une première face (face supérieure) de support sur laquelle sont fixées les deux diodes lumineuses. Sur sa face supérieure, le substrat 30 porte le deuxième photodétecteur PhD2, juxtaposé aux deux diodes D1, D2. Ce dernier devra être positionné de manière à capter une partie du rayonnement émis par chacune des deux diodes lumineuses. Le premier photodétecteur PhD1 est séparé des deux diodes lumineuses par un écran afin de ne pas capter le signal émis directement par les diodes lumineuses mais uniquement le signal transmis par les tissus environnants. Dans la variante des figures 5A à 5C, le premier photodétecteur PhD1 peut ainsi être fixé sur ladite première face, séparé des deux diodes lumineuses par un écran 32 opaque. Dans la variante des figures 6A à 6C, le premier photodétecteur PhD1 est fixé sur la face opposée (face inférieure) du substrat 30, l'écran étant alors formé par le substrat lui-même.

Pour rendre le bloc électronique hermétique, un capot 31 est apposé sur le substrat ; ce capot 31 sera totalement (ou localement) transparent.

Dans le cas où le premier photodétecteur PhD1 est fixé sur la face inférieure du substrat 30, un deuxième capot 310 peut être apposé pour recouvrir la photodiode. En variante, il est également possible de réaliser de remplacer le capot par un dépôt conforme de type ALD sur l'ensemble du bloc électronique, en dehors des plots de reprise des contacts électriques.

Selon les configurations, de manière non limitative, chaque substrat 30 peut être opaque ou au moins partiellement transparent pour laisser passer la lumière émise par les diodes lumineuses sans masquage inapproprié. Le positionnement du masquage dû aux pistes conductrices et l'étendue de transparence de la bague pourront être adaptés à l'orientation des diodes lumineuses D1, D2 et au niveau de diffusion de la lumière. Chaque module peut ainsi être configurée pour créer une illumination directive ou isotrope.

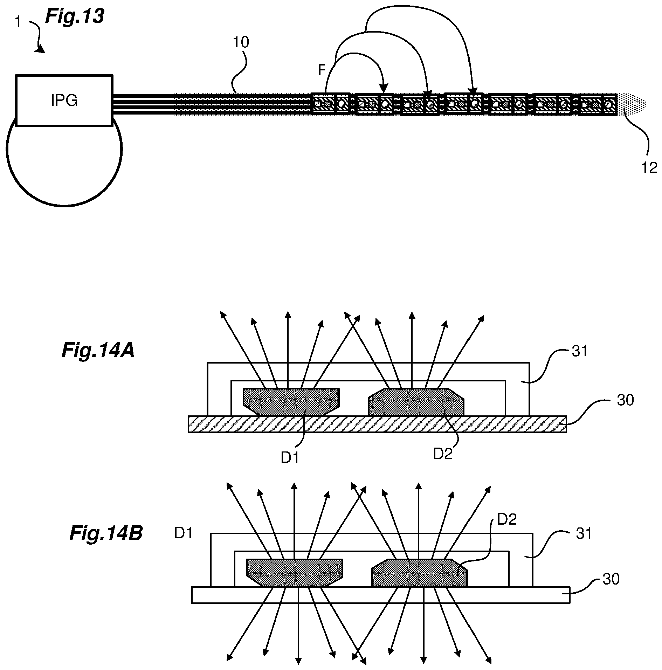

Les figures 14A et 14B illustrent différentes configurations possibles d'illumination. Sur la figure 14A, le module est à deux diodes fixées sur une même face du substrat 30 opaque. Le capot 31 est choisi transparent. L'illumination est ainsi réalisée que d'un seul côté. Sur la figure 14B, le substrat 30 est transparent et le capot 31 est également transparent, permettant d'émettre la lumière des deux côtés.

Les deux diodes lumineuses D1, D2 sont assemblées (par collage conducteur ou brasure) et intégrées dans le bloc électronique 3 hermétique.

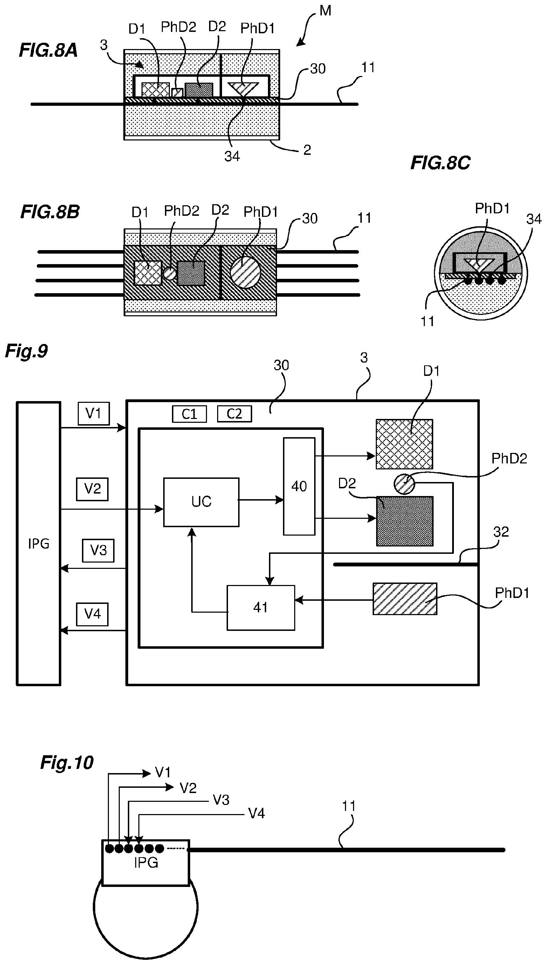

En référence à la figure 9, en plus des composants décrits ci-dessus, le bloc électronique 3 embarque une unité de commande et de traitement UC. Cette unité de commande et de traitement UC peut être un module CMOS. Elle est configurée pour remplir au moins les fonctions suivantes :
- Gestion de l'alimentation électrique des composants du bloc électronique 3 ;
- Décodage des signaux reçus en entrée ;
- Régulation de puissance pour alimenter les sources lumineuses du module ;
- Adressage des composants du bloc électronique 3 et gestion des fonctions ;
- Conditionnement des signaux fournis par les photodétecteurs PhD1, PhD2, le capteur de température, électrodes de mesure électrique (ECoG "Electrocorticogramme" - si présentes), du bloc électronique 3 (fonctions intégration, amplification, conversion courant-tension...) ;
- Protection contre les décharges électrostatiques ;

Le générateur IPG sur laquelle la sonde 10 est connectée est capable de fournir :
- des impulsions de faible tension (par exemple 0.1V, 40µsec) pour par exemple gérer les adresses des modules M et synchroniser les mesures si besoin ;
- des impulsions de "puissance" (par exemple 15V, 50mA, 250µsec) pour alimenter les diodes D1, D2, les condensateurs C1 et C2 et le bloc électronique 3 de chaque module M ;

Le générateur IPG intègre un microcontrôleur qui peut prendre en charge les différentes fonctions :
- Gestion d'alimentation du bloc électronique 3 de chaque module M de la sonde 10 ;
- Programmation journalière des différentes fonctions (illumination, type mesures, transfert signaux) ;
- Conversion (analogique vers donnée numérique correspondant à des valeurs physiques) ;
- Stockage des mesures réalisées ;
- Transfert des données à la demande ;

Le générateur IPG pourra être amené à effectuer un test de chaque module M, à des intervalles réguliers (programmés ou à la demande), pour vérifier son bon fonctionnement.

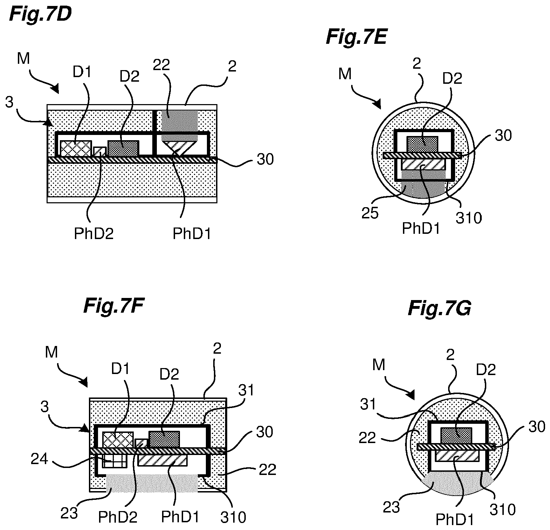

En référence aux figures 7A à 7F, le module peut présenter différentes fonctions supplémentaires, ces fonctions supplémentaires pouvant bien entendu se cumuler entre elles.

Figure 7A :
Le module de l'invention peut être de type hybride, c'est-à-dire portant des moyens de stimulation optique et des moyens de stimulation électrique. Dans ce cas, comme représenté sur la figure 7A, le module M, en plus de porter les diodes lumineuses D1, D2, portent également des électrodes 20 permettant une stimulation électrique des tissus environnants. Ces contacts électriques 20 peuvent être agencés sur au moins une partie de la surface de la paroi latérale du boîtier 2 en cylindre du module M. Les électrodes 20 peuvent être connectés au même générateur IPG que celui employé pour alimenter les diodes lumineuses et occuper certaines des voies spécifiques du générateur. Les électrodes peuvent être réalisée par exemple en Platine-Iridium massif ou par dépôt par pulvérisation ou en IrO2 avec dépôt localisé pour laisser passer le faisceau optique. Sur la figure 7A, à titre d'exemple, les électrodes 20 sont à section en arc de cercle et s'étendent sur une portion angulaire de la bague.

Lorsqu'un module est hybride, c'est-à-dire qu'il possède à la fois les moyens de stimulation optique et les moyens de stimulation électrique, il faut noter qu'il peut fonctionner selon l'un ou l'autre des deux modes de stimulation ou selon les deux modes de stimulation simultanément.

Figures 7B et 7C :
Le premier photodétecteur PhD1 peut être équipé de filtres sélectifs 21 (par exemple de type diélectrique) afin de permettre une mesure de fluorescence (par exemple des plaques amyloïdes marquées avec un fluorophore type "CRANADxxx").

Figures 7D et 7E :
Le premier photodétecteur PhD1 peut être équipé de guide d'onde optique 25 pour limiter la lumière parasite et optimiser la captation des photons.

Figures 7F et 7G :
Le module M peut comporter un hublot 23 transparent pour les sources de longueur d'onde supérieure à 4µm. Cet hublot est réalisé dans un autre matériau (silicium par exemple) que celui de son boîtier 2 (en saphir par exemple). Le bloc électronique peut alors intégrer un module FIR ("Far Infrared") 24 pour mesurer l'absorption des tissus dans la gamme de longueur d'onde 4-13µm.

L'unité de commande et de traitement UC peut comporter des composants de type analogique et/ou numérique.

Le bloc électronique peut également embarquer un circuit de pilotage 40 des deux diodes lumineuses et un circuit de traitement 41 des signaux fournis par les deux photodétecteurs PhD1, PhD2 et le capteur de température.

Plusieurs voies du générateur IPG peuvent être réservées pour gérer les fonctionnalités fournies par chaque module M.

Pour gérer les fonctionnalités d'un seul module, quatre conducteurs (par exemple) ou voies Vx (x allant de 1 à N voies du générateur) du générateur IPG sont nécessaires. En référence à la figure 10, pour chaque module, on peut ainsi disposer de l'architecture suivante :
- Une première voie V1 est destinée à l'alimentation du module : Le générateur peut fournir une tension d'environ 15V ;
- Une deuxième voie V2 est destinée à l'adressage du module ;
- Une troisième voie V3 permet de récupérer les signaux de mesure en provenance du module ;
- Une quatrième voie V4 sert pour la référence de tension ;

L'ensemble des voies du générateur IPG peuvent être réunies dans un câble 11 multibrins (4 à 12 voies par exemple). Les figures 8A à 8C illustrent un principe de connexion d'un module sur un câble 11 multibrins. Le câble 11 peut traverser chaque module M, sous le substrat 30 et chaque composant électronique du module M peut venir se connecter sur un conducteur du câble en employant une "via" 34 réalisée à travers le substrat 30.

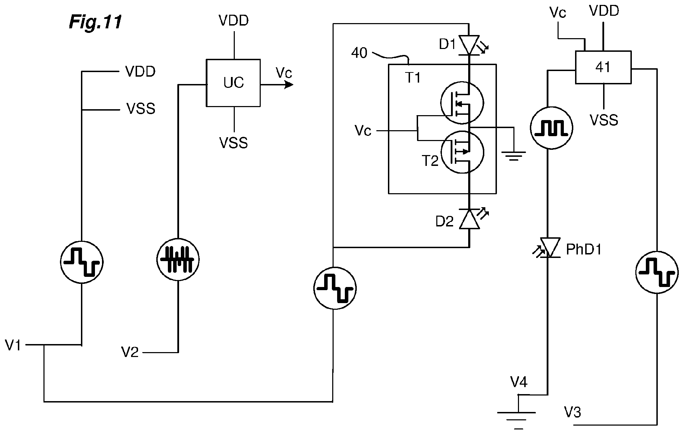

A titre d'exemple, la figure 11 montre l'architecture électronique et fonctionnelle d'un bloc électronique employé dans le module de l'invention. Sur cette figure 11, on a ainsi :
- La première voie V1 et la quatrième voie V4 destinées à l'alimentation de l'ensemble du bloc électronique 3 en fournissant les tensions d'alimentation VDD et VSS : le générateur IPG applique une tension d'alimentation (par exemple 15V sur cette voie) ;
- La deuxième voie V2 sur laquelle est connectée l'unité de commande et de traitement UC et sur laquelle peuvent être appliqués des signaux de commande ; l'unité de commande et de traitement UC peut comporter un décodeur destiné à décoder les signaux de commande reçus en entrée. En fonction des signaux reçus en entrée, l'unité de commande et de traitement UC est configurée pour générer en sortie un signal de pilotage Vc des diodes lumineuses D1, D2 à destination du circuit de pilotage 40 ; le circuit de pilotage 40 peut comporter deux transistors T1, T2 (par exemple de type MOSFET) connectés en série avec les LED, l'un de type p et l'autre de type n. Selon l'état du signal de pilotage Vc (polarité +/-) fourni par l'unité de commande (IPG) et de traitement UC et l'alternance de tension positive ou négative fournie par l'alimentation, le circuit de pilotage 40 est amené à allumer la première diode lumineuse D1 ou la deuxième diode lumineuse D2.
- La troisième voie V3 sur laquelle est connecté au moins un photodétecteur PhD1, câblé en sens direct ou inverse en fonction de l'électronique de mesure (photovoltaïque ou photoconducteur) du bloc électronique 3 et le circuit de traitement 41 des signaux fournis par ce photodétecteur PhD1 ; Le circuit de traitement 41 peut par exemple comporter un intégrateur, un convertisseur et tout autre composant classiquement employé dans le traitement de signal. Il faut noter que les signaux issus du photodétecteur PhD2 sont traités de la même façon. Ces mesures peuvent être réalisées à la demande, ou régulièrement selon un protocole préétabli et enregistré dans la mémoire embarquée dans le générateur (IPG). Ces points de mesure peuvent être réalisés en dehors de la phase d'illumination thérapeutique.

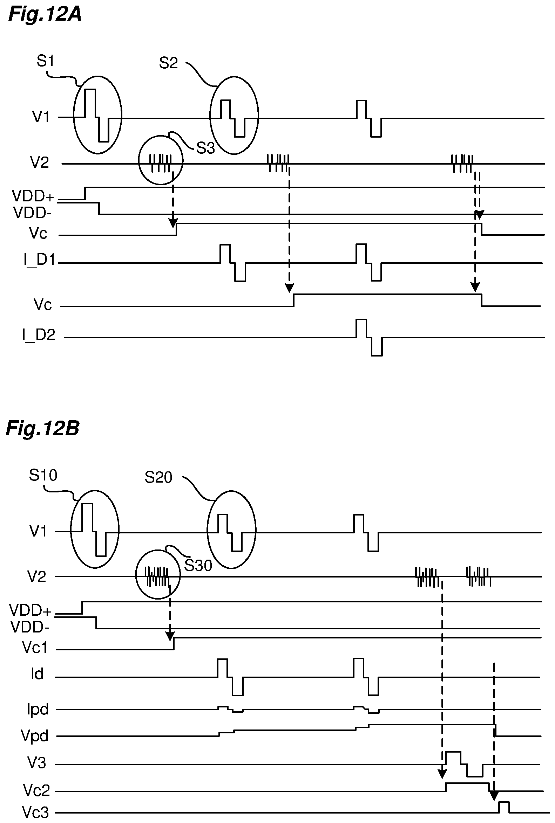

La figure 12A représente les chronogrammes illustrant le fonctionnement du dispositif dans un mode stimulation, c'est-à-dire en mode traitement des tissus. Sur cette figure 12A, on a ainsi :
- Les signaux d'alimentation fournis par le générateur sur sa première voie V1. Des premiers signaux S1 sont dédiés à l'alimentation des composants du bloc électronique et permettent de fournir la tension VDD (VDD+, VDD- ). Des deuxièmes signaux S2 sont dédiés à l'alimentation des diodes lumineuses D1, D2. Cette alimentation est pilotée selon l'état du signal de pilotage Vc fourni par l'unité de commande et de traitement UC.
- Les signaux d'adressage et de commande S3 fournis par le générateur IPG sur sa deuxième voie. Ces signaux S3 sont destinés à l'unité de commande et de traitement UC du module M. Lorsque le module M est adressé, son unité de commande et de traitement UC génère les signaux de pilotage Vc à destination du circuit de pilotage 40 des diodes lumineuses.
- Le niveau de courant I_D1, I_D2 qui traverse chacune des deux diodes lumineuses D1, D2, après commande.

La figure 12B représente les chronogrammes de commande et de mesure illustrant le principe de fonctionnement du dispositif dans un mode mesure. Sur la figure 12B, on a ainsi :
- Les signaux d'alimentation fournis par le générateur sur sa première voie V1. Des premiers signaux S10 sont dédiés à l'alimentation des composants du bloc électronique et permettent de fournir la tension VDD (VDD+, VDD- ). Des deuxièmes signaux S20 sont dédiés à l'alimentation des diodes lumineuses D1, D2. Cette alimentation est pilotée selon l'état du signal de pilotage Vc fourni par l'unité de commande et de traitement UC.
- Les signaux d'adressage et de commande S30 fournis par le générateur IPG sur sa deuxième voie. Ces signaux S30 sont destinés à l'unité de commande et de traitement UC du module M. Lorsque le module M est adressé, son unité de commande et de traitement UC génère les signaux de pilotage Vc (Vc1, Vc2 et Vc3 dans notre exemple) à destination du circuit de pilotage 40 des diodes lumineuses.
- Le niveau de courant Id qui traverse les deux diodes lumineuses D1, D2, après commande Vc1.
- Le signal de courant Ipd mesuré en sortie du photodétecteur PhD1 après commande Vc2.
- Le signal de tension Vpd obtenu en sortie du circuit de traitement 41.
- Le signal de tension qui est lu sur la troisième voie V3 par le générateur IPG
- La réinitialisation du photodétecteur et de l'intégrateur après réception du signal de commande Vc3.

Il faut noter que le dispositif conforme à l'invention, doté de plusieurs modules tels que décrits ci-dessus, sera à même de mettre en œuvre différents traitements ou différentes fonctions :
- Ajustement de la dose optique : Une séquence de traitement comporte la commande d'un premier module de la sonde en stimulation optique de manière à émettre un faisceau lumineux F et la mesure des signaux transmis par les tissus sur les autres modules de la sonde (voir figure 13). Cette séquence peut être répétée pour chaque module de la sonde. De telles mesures permettent au générateur de connaître la profondeur de pénétration de la lumière et ainsi les coefficients d'absorption/diffusion des tissus environnants, en vue d'ajuster la dose de lumière à émettre selon les résultats des mesures. De telles mesures peuvent être réalisées à intervalles réguliers ou en temps réel, en mettant en œuvre une boucle d'asservissement. La mesure effectuée sur le premier photodétecteur PhD1 de chaque module est proportionnelle à la profondeur de pénétration de lumière.
- Différents suivis biologiques :
   ∘ Micro-vascularisation pour évaluer le niveau de cicatrisation et/ou l'évolution des tissus environnants ;
   ∘ Mesure température, utile notamment en cas de maladie d'Alzheimer ;
   ∘ Contrôle de la densité des plaques amyloïdes au niveau de l'hippocampe ; Une mesure de DRS avec les longueurs d'onde adaptées permettra de voir s'il y a une évolution des tissus environnants ;
   ∘ Suivi du StO2 ("Cerebral Tissue Oxygen Saturation") ;
   ∘ Détection des plaques amyloïdes par mesure de florescence induite par un fluorophore de type CRANAD xxx par exemple ;

L'invention présente ainsi de nombreux avantages. On dispose d'un dispositif implantable de stimulation optique, "multiplexable" grâce à son unité de commande et de traitement UC et permettant une surveillance des tissus environnants grâce à l'emploi d'un photodétecteur.

L'avantage de réaliser une sonde d'illumination, implantée directement ou en contact des structures à traiter, permet d'avoir accès à l'évolution des paramètres biologiques directement sur les tissus concernés par mesure optique (DRS, amplitude pulsation, StO2...). Différentes sources de lumières de 650 à 1070nm sont connues pour être efficace dans le traitement des maladies neurodégénératives. Ces mêmes longueurs d'ondes sont parfaitement adaptées pour les mesures de vascularisation, de saturation O2, d'absorption et diffusion des tissus cérébraux.

Ce dispositif permet par ailleurs d'enregistrer les paramètres optiques des tissus pendant la mise en place de la sonde. En particulier :
- Relever les paramètres optiques (absorption et diffusion par mesure DRS) pour différentes longueurs d'ondes afin d'optimiser la dose appliquée au patient par stimulation ;
- Repérer les tissus, par simple mesure de réflectivité, pour aider au placement en profondeur de la sonde (exemple dans le cas de la maladie de parkinson, des électrodes sont placées dans la STN pour compenser le manque de dopamine et la sonde se prolonge sur 5 à 10 mm pour illuminer la SNc, noyau visé par le traitement NIR) ;
- Surveiller les micros hémorragies pendant la descente de la sonde ;

Le dispositif est compatible avec l'emploi d'un générateur IPG classique. Il est par ailleurs particulièrement compact, adapté à une chirurgie profonde et peut s'avérer particulièrement polyvalent grâce à la possibilité de stimulation optique et électrique.

En outre, il faut noter que le dispositif peut être fabriqué à partir de technologies standards :
- Technologie CMOS pour le bloc électronique ;
- Technologie de type LED, OLED, µLED, VCSEL, Laser pour les diodes ;
- Photodétecteur en silicium ou matrice de photodiodes CMOS ;
- Electrodes métalliques sous forme de bagues ou de lames ;
- Herméticité par soudure laser du capot et/ou dépôt ALD ("Atomic Layer Deposit") sur des composants du bloc électronique ;

## Revendications

1. Module (M) à stimulation optique à intégrer dans une sonde implantable dans un être vivant pour illuminer de manière localisée une zone dudit être vivant, **caractérisé en ce qu'**il comporte :
- Un boîtier (2) au moins en partie transparent,
- Un bloc (3) électronique hermétique placé dans ledit boîtier et comportant un volume interne logeant une source lumineuse (D1, D2), une unité de commande et de traitement (UC), un premier photodétecteur (PhD1),
- Un dispositif de connexion pour raccorder ledit module à une unité centrale d'alimentation et de commande,
**caractérisé en ce que** ledit bloc comporte, en outre :
un deuxième photodétecteur (PhD2), un écran opaque séparant son volume interne en un premier espace contenant la source lumineuse et le deuxième photodétecteur (PhD2), et un deuxième espace contenant le premier photodétecteur (PhD1).

2. Module selon la revendication 1, comportant un filtre optique (21) de sélection de longueurs d'ondes appliqué audit premier photodétecteur (PhD1).

3. Module selon la revendication 1 ou 2, comportant au moins un guide d'ondes (25) agencé sur le premier photodétecteur (PhD1) en vue de limiter toute lumière parasite et d'optimiser une captation de photons.

4. Module selon l'une des revendications 1 à 3, comportant un hublot (23) transparent, choisi pour capter des signaux de longueur d'onde supérieure à 4µm.

5. Module selon l'une des revendications 1 à 4, ledit bloc électronique comportant au moins un substrat (30) comportant deux faces opposées, lesdites deux sources lumineuses étant montées sur une seule des deux faces dudit substrat (30).

6. Module selon la revendication 5, ledit bloc électronique (3) comportant un capot hermétique (31) adapté sur le substrat (30) et formant ledit volume interne du bloc électronique.

7. Module selon l'une des revendications 1 à 6, ledit boîtier (2) comportant une bague fermée à ses deux extrémités par deux bouchons, lesdits deux bouchons portant des moyens de support du bloc électronique (3) logé dans le boîtier.

8. Module selon la revendication 7, comportant des électrodes (20) de stimulation électrique sur la surface latérale de sa bague.

9. Module selon l'une des revendications 1 à 8, comportant un matériau d'enrobage (22) injecté dans son boîtier (2) autour du bloc électronique (3) hermétique.

10. Sonde implantable dans un être vivant destinée à être connectée électriquement à une source d'alimentation électrique et présentant une architecture allongée, comportant plusieurs modules (M) à stimulation optique tels que définis dans l'une des revendications 1 à 9, lesdits modules étant juxtaposés le long de la sonde et séparés entre eux d'une distance non nulle, ladite sonde comprenant un matériau d'enrobage (12) venant combler l'espace entre deux modules adjacents.

11. Dispositif (1) d'illumination implantable, destiné à être implanté dans un être vivant pour illuminer de manière localisée une zone dudit être vivant, ledit dispositif comportant une source d'alimentation électrique comportant plusieurs voies (Vx) d'alimentation électrique en parallèle et une sonde (10) connectée électriquement à la source d'alimentation électrique et présentant une architecture allongée entre une extrémité proximale et une extrémité distale, ladite sonde (10) étant telle que définie dans la revendication 10.

12. Dispositif selon la revendication 11, ladite source d'alimentation étant un générateur d'impulsions implantable (IPG).

13. Dispositif selon la revendication 11 ou 12, comportant des moyens de détermination d'une dose de lumière à appliquer en fonction de signaux reçus en provenance d'un ou plusieurs premiers photodétecteurs des modules (M).

## Patentansprüche

1. Modul (M) mit optischer Stimulation zum Einbau in eine in ein Lebewesen implantierbare Sonde, um einen Bereich des Lebewesens örtlich begrenzt zu beleuchten, **dadurch gekennzeichnet, dass** es aufweist:
- ein zumindest teilweise transparentes Gehäuse (2),
- einen hermetisch dichten Elektronikblock (3), der im Gehäuse platziert ist und ein Innenvolumen aufweist, in dem eine Lichtquelle (D1, D2), eine Steuer- und Verarbeitungseinheit (UC), ein erster Fotodetektor (PhD1) untergebracht sind,
- eine Verbindungsvorrichtung, um das Modul an eine zentrale Versorgungs- und Steuereinheit anzuschließen,
**dadurch gekennzeichnet, dass** der Block außerdem aufweist:
- einen zweiten Fotodetektor (PhD2), einen lichtundurchlässigen Schirm, der sein Innenvolumen in einen die Lichtquelle und den zweiten Fotodetektor (PhD2) enthaltenden ersten Raum und einen den ersten Fotodetektor (PhD1) enthaltenden zweiten Raum trennt.

2. Modul nach Anspruch 1, das ein optisches Filter (21) zur Auswahl von Wellenlängen aufweist, das an den ersten Fotodetektor (PhD1) angewendet wird.

3. Modul nach Anspruch 1 oder 2, das mindestens einen Wellenleiter (25) aufweist, der auf dem ersten Fotodetektor (PhD1) eingerichtet ist, um jedes Streulicht zu begrenzen und einen Photoneneinfang zu optimieren.

4. Modul nach einem der Ansprüche 1 bis 3, das ein transparentes Sichtfenster (23) aufweist, das ausgewählt wird, um Signale einer Wellenlänge von mehr als 4µm aufzufangen.

5. Modul nach einem der Ansprüche 1 bis 4, wobei der Elektronikblock mindestens ein Substrat (30) aufweist, das zwei gegenüberliegende Seiten aufweist, wobei die zwei Lichtquellen auf eine einzige der zwei Seiten des Substrats (30) montiert sind.

6. Modul nach Anspruch 5, wobei der Elektronikblock (3) eine hermetisch dichte Haube (31) aufweist, die auf dem Substrat (30) angepasst ist und das Innenvolumen des Elektronikblocks bildet.

7. Modul nach einem der Ansprüche 1 bis 6, wobei das Gehäuse (2) einen an seinen zwei Enden durch zwei Verschlusskappen verschlossenen Ring aufweist, wobei die zwei Verschlusskappen Stützeinrichtungen des im Gehäuse untergebrachten Elektronikblocks (3) tragen.

8. Modul nach Anspruch 7, das Elektroden (20) zur elektrischen Stimulation auf der Seitenfläche seines Rings aufweist.

9. Modul nach einem der Ansprüche 1 bis 8, das ein Ummantelungsmaterial (22) aufweist, das in sein Gehäuse (2) um den hermetisch dichten Elektronikblock (3) herum eingespritzt wird.

10. In ein Lebewesen implantierbare Sonde, die dazu bestimmt ist, elektrisch mit einem Stromversorgungsgerät verbunden zu werden und eine längliche Architektur aufweist, die mehrere Module (M) mit optischer Stimulation wie in einem der Ansprüche 1 bis 9 definiert aufweist, wobei die Module entlang der Sonde nebeneinander angeordnet und voneinander durch einen Abstand ungleich Null getrennt sind, wobei die Sonde ein Ummantelungsmaterial (12) enthält, das den Raum zwischen zwei benachbarten Modulen ausfüllt.

11. Implantierbare Beleuchtungsvorrichtung (1), die dazu bestimmt ist, in ein Lebewesen implantiert zu werden, um einen Bereich des Lebewesens örtlich begrenzt zu beleuchten, wobei die Vorrichtung ein mehrere parallele Stromversorgungskanäle (Vx) aufweisendes Stromversorgungsgerät und eine Sonde (10) aufweist, die elektrisch mit dem Stromversorgungsgerät verbunden ist und eine längliche Architektur zwischen einem proximalen und einem distalen Ende aufweist, wobei die Sonde (10) wie in Anspruch 10 definiert ist.

12. Vorrichtung nach Anspruch 11, wobei das Versorgungsgerät ein implantierbarer Impulsgenerator (IPG) ist.

13. Vorrichtung nach Anspruch 11 oder 12, die Einrichtungen zur Bestimmung einer abhängig von von einem oder mehreren ersten Fotodetektoren der Module (M) empfangenen Signalen anzuwendenden Lichtdosis aufweist.

## Claims

1. Optically stimulating module (M) to be integrated into a probe that is implantable into a living being with a view to locally illuminating a region of said living being, **characterized in that** it comprises:
- a casing (2) that is at least partially transparent,
- a hermetic electronic unit (3) that is placed in said casing and that comprises an internal volume housing a light source (D1, D2), a control and processing unit (UC), and a first photodetector (PhD1),
- a connecting device for connecting said module to a central control and power unit.
**characterized in that** said unit further comprises: a second photodetector (PhD2), an opaque screen separating its internal volume into a first space containing the light source and the second photodetector (PhD2), and a second space containing the first photodetector (PhD1).

2. Module according to Claim 1, comprising a wavelength-selecting optical filter (21) applied to said first photodetector (PhD1).

3. Module according to Claim 1 or 2, comprising at least one waveguide (25) arranged on the first photodetector (PhD1) with a view to limiting any parasitic light and to optimizing a capture of photons.

4. Module according to one of Claims 1 to 3, comprising a transparent porthole (23), chosen to capture signals of wavelength longer than 4 µm.

5. Module according to one of Claims 1 to 4, said electronic unit comprising at least one substrate (30) comprising two opposite faces, said two light sources being mounted on a single of the two faces of said substrate (30).

6. Module according to Claim 5, said electronic unit (3) comprising a suitable hermetic cover (31) on the substrate (30), said cover forming said internal volume of the electronic unit.

7. Module according to one of Claims 1 to 6, said casing (2) comprising a ring that is closed at its two ends by two plugs, said two plugs bearing means for holding the electronic unit (3) housed in the casing.

8. Module according to Claim 7, comprising electrically stimulating electrodes (20) on the lateral surface of its ring.

9. Module according to one of Claims 1 to 8, comprising a coating material (22) injected into its casing (2) around the hermetic electronic unit (3).

10. Probe that is implantable into a living being, said probe being intended to be electrically connected to an electrical power source and having an elongate architecture, comprising a plurality of optically stimulating modules (M) such as defined in one of Claims 1 to 9, said modules being juxtaposed along the probe and separated from one another by a nonzero distance, said probe comprising a coating material (12) filling the space between two adjacent modules.

11. Implantable illuminating device (1) intended to be implanted into a living being with a view to locally illuminating a region of said living being, said device comprising an electrical power source comprising a plurality of parallel electrical supply paths (Vx) and a probe (10) that is electrically connected to the electrical power source and that has an elongate architecture between a proximal end and a distal end, said probe (10) being such as defined in Claim 10.

12. Device according to Claim 11, said power source being an implantable pulse generator (IPG).

13. Device according to Claim 11 or 12, comprising means for determining a light dose to be applied depending on received signals coming from one or more first photodetectors of the modules (M).
